(19) Europäisches Patentamt
European Patent Office
Office européen des brevets

(11) **EP 4 299 182 A1**

(12) **EUROPEAN PATENT APPLICATION**
published in accordance with Art. 153(4) EPC

(43) Date of publication:
**03.01.2024 Bulletin 2024/01**

(21) Application number: **22780418.4**

(22) Date of filing: **23.03.2022**

(51) International Patent Classification (IPC):
**B02C 13/14** (2006.01)    **B02C 19/18** (2006.01)
**A61L 27/22** (2006.01)    **A61L 27/54** (2006.01)
**A61L 27/56** (2006.01)    **C07K 14/00** (2006.01)

(52) Cooperative Patent Classification (CPC):
**B02C 13/14; B02C 19/18; C07K 14/00;** A61L 27/22;
A61L 27/54; A61L 27/56

(86) International application number:
**PCT/JP2022/013691**

(87) International publication number:
**WO 2022/210204 (06.10.2022 Gazette 2022/40)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.03.2021   JP 2021060910**

(71) Applicant: **FUJIFILM Corporation
Tokyo 106-8620 (JP)**

(72) Inventors:
• **FUKUNAGA, Kazuto
Ashigarakami-gun, Kanagawa 258-8577 (JP)**
• **KATO, Nobuhiko
Ashigarakami-gun, Kanagawa 258-8577 (JP)**

(74) Representative: **Hoffmann Eitle
Patent- und Rechtsanwälte PartmbB
Arabellastraße 30
81925 München (DE)**

Remarks:
The complete document including Reference Table(s) and the Sequence Listing(s) can be downloaded from the EPO website

(54) **CRUSHING METHOD, POLYMER BLOCK MANUFACTURING METHOD, AND CRUSHING DEVICE**

(57)    Provided is a pulverizing method including a pulverizing step of pulverizing a polymer porous body and an electricity removing step of removing electricity from the polymer porous body during the pulverization in the pulverizing step.

EP 4 299 182 A1

# FIG. 1

**Description**

BACKGROUND OF THE INVENTION

1. Field of the Invention

The present disclosure relates to a pulverizing method, a method for producing a polymer block, and a pulverization device.

2. Description of the Related Art

Currently, a practical application of regenerative medicine for regeneration of biological tissues and organs, which have been dysfunctional or malfunctioning, has progressed. In biological tissues which cannot be recovered by natural healing ability of the living body alone, the regenerative medicine is a medical technology that recreates the same form and function as the original tissue using three factors of cells, scaffolds, and growth factors. Among these, bone regeneration in the orthopedic field or the dental field has been known as one of fields attracting attention in the regenerative medicine field. In a case where the bone disease is in legs and lower back, it is not possible to walk due to a defect caused by the disease, and in a case of dentistry, since it is difficult to eat, the bone disease causes a marked deterioration in quality of life (QOL). In the field of regenerative medicine, collagen or gelatin having high biocompatibility is used as a base material. For example, WO2014/133081A discloses a biocompatible polymer block obtained by pulverizing a porous body of recombinant gelatin.

In addition, in the pulverization of the polymer porous body, there is a problem that a polymer block after the pulverization is burnt due to frictional heat generated during the pulverization. As a technique for suppressing the burning, JP1980-092667A (JP-S55-092667A) discloses a method of performing pulverization at an ultra-low temperature using liquid nitrogen. In addition, JP 1997-000176A (JP-H9-000176A) discloses a method of mixing with a liquid and performing wet pulverization.

SUMMARY OF THE INVENTION

However, the pulverization at an ultra-low temperature, as disclosed in JP1980-092667A (JP-S55-092667A), has a problem that the cost for cooling increases. In addition, the wet pulverization as disclosed in JP 1997-000176A (JP-H9-000176A) has a problem that it takes time and effort to dry the liquid for separating and removing the liquid. In recent years, there has been a demand for a new method for pulverizing the polymer porous body capable of suppressing the occurrence of burning, which can be substituted for such a method or can be used in combination with such a method.

The present disclosure provides a pulverizing method that can suppress burning which occurs in a case of pulverizing a polymer porous body, a method for producing a polymer block, and a pulverization device.

A first aspect of the present disclosure is a pulverizing method including a pulverizing step of pulverizing a polymer porous body and an electricity removing step of removing electricity from the polymer porous body during the pulverization in the pulverizing step.

According to a second aspect of the present disclosure, in the electricity removing step of the above-described first aspect, the electricity removal may be performed on the polymer porous body by irradiating the polymer porous body with a radiation.

According to a third aspect of the present disclosure, in the above-described second aspect, the radiation may be at least one of X-rays or ultraviolet rays.

According to a fourth aspect of the present disclosure, in the above-described third aspect, the radiation may be X-rays having a tube voltage of a radiation source of 4 kV or more and 50 kV or less.

According to a fifth aspect of the present disclosure, in the electricity removing step of the second aspect to fourth aspect described above, a 70 $\mu$m-dose equivalent rate of the radiation radiated to the polymer porous body may be 1 mSv/h or more and 200 Sv/h or less.

According to a sixth aspect of the present disclosure, in the electricity removing step of the above-described aspects, an electricity removing time until a potential in at least a part of a pulverizing portion where the polymer porous body is pulverized changes from +1000 V to +100 V may be 0.01 seconds or more and 10 seconds or less.

According to a seventh aspect of the present disclosure, in the pulverizing step of the above-described aspects, the polymer porous body may be pulverized using a pulverizing portion which performs dry pulverization.

According to an eighth aspect of the present disclosure, in the above-described seventh aspect, the pulverizing portion may include a screen and a rotary impeller.

According to a ninth aspect of the present disclosure, in the seventh aspect or eighth aspect described above, the pulverizing portion may be ventilated at a ventilation rate of $1 \times 10^4$ times/h or more and $1 \times 10^7$ times/h or less.

According to a tenth aspect of the present disclosure, in the electricity removing step of the above-described ninth aspect, the electricity removal may be performed on the polymer porous body by irradiating at least a part of the pulverizing portion with a radiation.

According to an eleventh aspect of the present disclosure, the polymer porous body may contain a protein.

According to a twelfth aspect of the present disclosure, in the above-described eleventh aspect, the polymer porous body may contain the following peptide (A), (B), or (C).

(A) a peptide consisting of an amino acid sequence set forth in SEQ ID NO: 1

(B) a peptide consisting of an amino acid sequence in which one or several amino acid residues in the amino acid sequence set forth in SEQ ID NO: 1 are modified, and having biocompatibility

(C) a peptide consisting of an amino acid sequence which has a partial sequence having 80% or more sequence identity with a partial amino acid sequence consisting of 4th to 192nd amino acid residues in the amino acid sequence set forth in SEQ ID NO: 1, and having biocompatibility

According to a thirteenth aspect of the present disclosure, in the above-described aspects, a size of the polymer porous body before the pulverization may be 0.1 mm or more and 50 mm or less.

According to a fourteenth aspect of the present disclosure, in the above-described aspects, a size of the polymer porous body after the pulverization may be 0.01 mm or more and 10 mm or less.

A fifteenth aspect of the present disclosure is a method for producing a polymer block, including a pulverizing step of pulverizing a polymer porous body and an electricity removing step of removing electricity from the polymer porous body during the pulverization in the pulverizing step.

A sixteenth aspect of the present disclosure is a pulverization device including a pulverizing portion which pulverizes a polymer porous body and an irradiating portion which irradiates at least a part of the pulverizing portion with a radiation, in which electricity is removed from the polymer porous body during the pulverization in the pulverizing portion by the radiation radiated from the irradiating portion.

According to a seventeenth aspect of the present disclosure, in the above-described sixteenth aspect, the pulverization device further includes a ventilating portion which ventilates the pulverizing portion, in which a ventilation rate of the pulverizing portion by the ventilating portion may be $1 \times 10^4$ times/h or more and $1 \times 10^7$ times/h or less.

In the present specification, the term "step" includes not only the independent step but also a step in which intended purposes are achieved even in a case where the step cannot be precisely distinguished from other steps.

According to the above-described aspects, with the pulverizing method, the method for producing a polymer block, and the pulverization device of the present disclosure, it is possible to suppress burning which occurs in a case of pulverizing a polymer porous body.

BRIEF DESCRIPTION OF THE DRAWINGS

Fig. 1 is a schematic configuration view showing an example of a configuration of a pulverization device.
Fig. 2 is a characteristic curve of an X-ray film.
Fig. 3 is a schematic configuration view showing another example of a configuration of a pulverization device.

DESCRIPTION OF THE PREFERRED EMBODIMENTS

Hereinafter, embodiments according to the technique of the present disclosure will be described in detail.

[Method of pulverizing polymer porous body]

A method of pulverizing a polymer porous body according to the present exemplary embodiment includes a pulverizing step of pulverizing a polymer porous body and an electricity removing step of removing electricity from the polymer porous body during the pulverization in the pulverizing step. Hereinafter, the polymer porous body after being pulverized by the pulverizing step is referred to as "polymer block". In other words, the method according to the present exemplary embodiment is a method for producing a polymer block by performing a pulverizing step of pulverizing a polymer porous body and an electricity removing step of removing electricity from the polymer porous body during the pulverization in the pulverizing step. Hereinafter, the pulverizing method according to the present exemplary embodiment and the method for producing a polymer block are collectively referred to as "method according to the present disclosure". In the method according to the present disclosure, after the pulverizing step and the electricity removing step, the pulverizing step and the electricity removing step may be repeated again. The number of times of performing the pulverizing step and the electricity removing step is preferably 1 to 4, and more preferably 2 or 3.

(Pulverizing step)

As described above, the method according to the present disclosure includes a pulverizing step of pulverizing a polymer porous body. In the present specification, the "pulverization" means fragmentation of solids and aggregates of solids by application of mechanical energy. Examples of a pulverization unit in the method according to the present disclosure include dry pulverization and wet pulverization, and from the viewpoint of saving time, effort, and cost for drying for separating and removing the liquid, it is preferable to use dry pulverization. The pulverization can be performed by a pulverizing portion (details will be described later) provided in a pulverization device.

(Electricity removing step)

As described above, the method according to the present disclosure includes an electricity removing step of removing electricity from the polymer porous body during the pulverization in the above-described pulverizing step. In the present specification, the "electricity removing" means removing static electricity from a charged substance. Examples of an electricity removing method in the method according to the present disclosure include a radiation irradiation method, a corona discharge method, an antistatic agent addition method, and a humidification method. In addition, two or more of these electricity removing methods may be appropriately combined. Among these, from the viewpoint of eliminating the need for the addition of the antistatic agent or the like, and the formation of a high-humidity environment which may affect a friction coefficient of a material to be pulverized, a radiation irradiation method or a corona discharge method is preferable. In particular, according to the radiation irradiation method, electricity removing capacity can be enhanced. The irradiation of radiation can be performed by an irradiating portion (details will be described later) provided in a pulverization device.

The "radiation" which is radiated to the polymer porous body in the electricity removing step includes particle beams such as $\alpha$-rays and $\beta$-rays, electromagnetic waves such as X-rays and $\gamma$-rays, and non-ionizing radiation such as infrared rays, visible rays, and ultraviolet rays. However, from the viewpoint that it is difficult to cause denaturation of the polymer porous body to be irradiated, it is preferable that the radiation is at least one of X-rays or ultraviolet rays. In addition, from the viewpoint that air inside the polymer porous body can be ionized due to permeability and the electricity removal can also be performed from the inside of the polymer porous body, X-rays are more preferable. In addition, from the viewpoint of preventing the denaturation of the polymer porous body, X-rays having a tube voltage of a radiation source of 4 kV or more and 50 kV or less (so-called soft X-rays) are still more preferable, and X-rays having a tube voltage of a radiation source of 4 kV or more and 20 kV or less are particularly preferable.

A 70 $\mu$m-dose equivalent rate of the above-described radiation is preferably 1 mSv/h or more and 200 Sv/h or less, and more preferably 2 mSv/h or more and 200 Sv/h or less. The irradiation dose of the radiation can be measured by, for example, a survey meter. In addition, the 70 $\mu$m-dose equivalent rate of the radiation is a value measured based on a method described in (Measurement of irradiation dose) in Example 1 described later. In the electricity removing step, it is preferable that the radiation is continuously radiated during the pulverization of the polymer porous body in the pulverizing step.

Electricity removing ability can be evaluated using a charge plate monitor (also referred to as a charged plate monitor, an ionizer performance tester, or the like). For example, a charge plate monitor in accordance with ANSI/ESD-STM 3.1, such as MODEL 156A (manufactured by TREK), or the like can be used. In a charged electrode having a specific capacitance, a time required to change from a specific initial potential to a specific potential due to electricity removing is called an electricity removing time, and by measuring this, the time required for electricity removing can be evaluated. For example, in 1 inch-square charge plate (156P-C25X25-S3M manufactured by TREK) with a capacitance of 20 $\pm$ 1 picofarad, a time required to change from an initial potential of +1000 V to a potential of +100 V can be measured as the electricity removing time. The electricity removing time measured by the method is preferably 0.01 seconds or more and 10 seconds or less, and more preferably 0.01 seconds or more and 2 seconds or less in at least a part of the pulverizing portion in the pulverization device.

(Classifying step)

The method according to the present disclosure may further include a classifying step of classifying the polymer block (the polymer porous body after the pulverization). The "classification" is an operation of classifying polymer blocks having different properties according to their properties. Examples of the properties of the polymer block include size, shape, density, magnetism, color, and chemical components. Examples of the classifying method by the size of the polymer block include fluid classification performed with a fluid and sieve separation in which a sieve is used to separate polymer blocks larger than a mesh of the sieve and polymer blocks smaller than that. A cyclone may be applied as the fluid classification. A sieve, a mesh, a filter, or the like may be applied as the sieve separation. The classifying step may be performed simultaneously with the pulverizing step and/or the electricity removing step, or may be performed after the pulverizing step and/or the electricity removing step. The classifying step by the sieve separation can be performed by a sieve for classifying (details will be described later) provided in a pulverization device.

According to the method according to the present disclosure, including each of the steps described above, it is possible to suppress burning due to frictional heat generated during the pulverization of the polymer porous body. The present inventors have presumed this factor as follows.

The polymer porous body during the pulverization may be charged and adhered to the pulverizing portion due to static electricity generated by friction with the pulverizing portion and/or other polymer porous bodies. In a case where the polymer porous body during the pulverization is charged and adhered to the pulverizing portion, since friction time increases, the amount of frictional heat also increases and the burning is likely to occur. The "friction time" here means a time in which the polymer porous body during the pulverization receives frictional force due to the friction with the pulverizing portion and/or other polymer porous bodies.

Since the electrostatic adhesion is determined by a difference between force of adhesion due to electrostatic force and force of detachment due to gravity, as a density of an object is smaller, the force of detachment is smaller than the force of adhesion, and the electrostatic adhesion is likely to occur. Therefore, the polymer porous body, which has a density lower than that of a non-porous polymer, is likely to be charged and adhered to the pulverizing portion, and is likely to be burnt. The "burning" means that, oxidation, decomposition, crosslinking, Maillard reaction, and/or the like occur in the polymer porous body due to temperature rise by the frictional heat, and the properties change (for example, coloration).

Therefore, in the method according to the present disclosure, the electricity is removed from the polymer porous body during the pulverization in the pulverizing portion. As a result, it is presumed that the electrostatic adhesion of the polymer porous body in the pulverizing portion can be suppressed, and the friction time can be shortened to reduce the amount of the frictional heat. Accordingly, it is possible to suppress the occurrence of burning, even in the pulverization of the polymer porous body which is easily charged and adhered due to the low density so that the burning is likely to occur.

[Pulverization device]

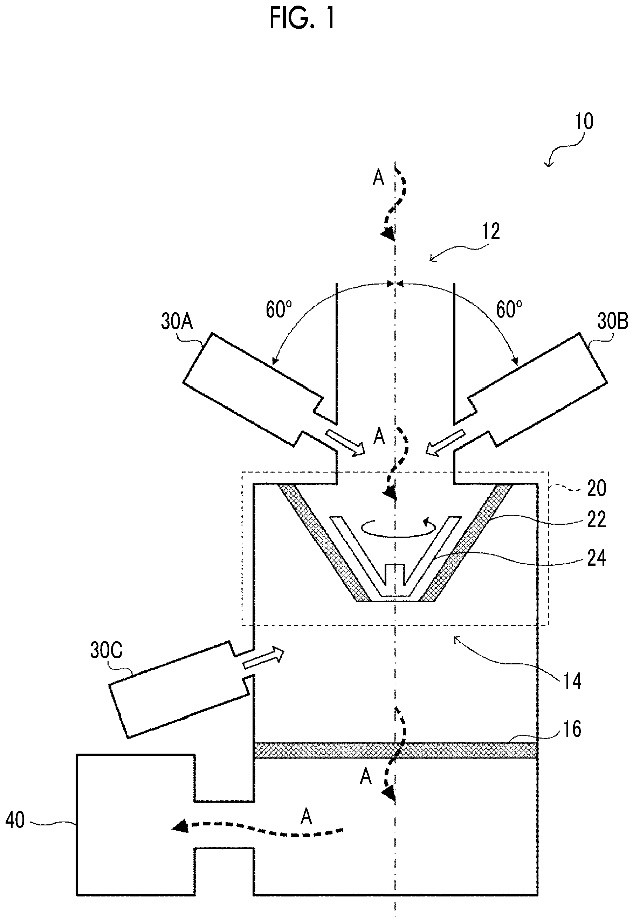

Next, a pulverization device as another exemplary embodiment of the present disclosure will be described. An example of a pulverization device 10 according to the present exemplary embodiment will be described with reference to Fig. 1. Fig. 1 is a schematic view showing a configuration of the pulverization device 10. The pulverization device 10 has a function of executing the pulverizing step, electricity removing step, and classifying step in the method according to the exemplary embodiment described above.

As shown in Fig. 1, the pulverization device 10 includes a pulverizing portion 20, at least one irradiating portion 30, and a ventilating portion 40. In Fig. 1, three irradiating portions 30A to 30C are illustrated as an example of the irradiating portion 30, but hereinafter, in a case where the irradiating portions 30A to 30C are not distinguished, they are simply referred to as "irradiating portion 30". In the pulverization device 10, in a case where the polymer porous body is put into an inlet 12, the polymer porous body is pulverized by the pulverizing portion 20, and then ejected from an ejecting portion 14. Each arrow A in Fig. 1 indicates a flow of air ventilated by the ventilating portion 40, and the polymer porous body is also transported along this flow.

The pulverizing portion 20 pulverizes the polymer porous body. As the pulverizing portion 20, a pulverizing function included in various known devices for performing the dry pulverization and the wet pulverization can be applied. Examples of such a device include a screen mill, a hammer mill, a ball mill, a beads mill, a jet mill, a cutter mill, a vibration mill, a roller mill, a line mill, a stamp mill, a disc mill, a pin mill, a grinding mill, a rotor mill, a cyclone mill, a rod mill, a power mill, a pot mill, a jaw crusher, a gyre crusher, an impact crusher, a roll crusher, and an edge runner. Among these, from the viewpoint of saving time, effort, and cost for drying for separating and removing the liquid, it is preferable to use a device for performing the dry pulverization.

In particular, from the viewpoint that variation in particle size distribution is small by collecting at any time from those pulverized to a certain size, it is preferable to use a screen mill which is an example of the device for performing the dry pulverization. Examples of the screen mill include Comil manufactured by Quadro Engineering. Fig. 1 shows a view in which a pulverizing function included in the screen mill is used as the pulverizing portion 20. The screen mill includes a screen 22 provided with a plurality of holes and a rotary impeller 24 as the pulverizing function. In the screen mill, by pressing the polymer porous body put from the inlet 12 against the screen 22 using the rotary impeller 24, the polymer porous body is pulverized to a size equal to or smaller than a hole size of the screen 22, and then ejected from the ejecting portion 14. The rotary impeller is preferably of a rake type, a round shape, or a square shape, and more preferably of a rake type. In Fig. 1, illustration of a motor or the like, which drives the rotary impeller 24, is omitted.

From the viewpoint of setting a size of the polymer block to a size suitable for a tissue repair material (details will be described later), a hole diameter of the screen 22 is preferably 0.006 inch or more and 0.25 inch or less, and more preferably 0.04 inch or more and 0.08 inch or less. From the viewpoint of efficient pulverization, a rotation speed of the rotary impeller is preferably 100 rpm or more and 6000 rpm or less, and more preferably 1000 rpm or more and 3000 rpm or less. A wind speed in the holes of the screen 22 is preferably 5 m/s or more and 20 m/s or less, and more preferably 7 m/s or more and 15 m/s or less.

In the present specification, the pulverizing portion 20 refers to a region where the polymer porous body can be actually pulverized, and for example, in the case of Fig. 1, it refers to a region of a trapezoidal rotor inside the screen 22. In a case where a known screen mill is applied in the pulverization device 10, the screen 22 and the rotary impeller 24 included in the screen mill correspond to the pulverizing portion 20, and other constituent elements included in the screen mill do not correspond to the pulverizing portion 20. For example, an inlet and an ejecting portion included in a known screen mill each correspond to the inlet 12 and the ejecting portion 14 in the pulverization device 10.

In the irradiating portion 30, by irradiating at least a part of the pulverizing portion 20 with radiation, the polymer porous body during the pulverization in the pulverizing portion 20 is irradiated with radiation to remove electricity of the polymer porous body. By irradiating at least a part of the pulverizing portion 20 with radiation, the air in the pulverizing portion 20 is ionized, and positive and negative ions are generated. That is, ions having a charge opposite to the charge of the polymer porous body charged by the static electricity are generated. As a result, the charge of the polymer porous body is neutralized, and the electricity of the polymer porous body is removed. Since the polymer porous body is stirred in the pulverizing portion 20, in a case where the electricity of the polymer porous body is removed in the at least a part of the pulverizing portion 20, it is possible to obtain an effect of suppressing the occurrence of burning.

In addition, in the irradiating portion 30, in addition to the at least a part of the pulverizing portion 20, the inlet 12 and the ejecting portion 14 may be irradiated with radiation. By irradiating the inlet 12 and the ejecting portion 14 with radiation, it is possible to suppress the electrostatic adhesion of the polymer porous body and the polymer block in the inlet 12 and the ejecting portion 14, so that the yield of the polymer block can be improved.

A position of the irradiating portion 30 in the pulverization device 10 is not particularly limited as long as it is a position where the at least a part of the pulverizing portion 20 can be irradiated with radiation by at least one of the irradiating portions 30. However, at least one of the irradiating portions 30 is preferably provided at an angle of 30 to 90 degrees with respect to a vertical direction of the pulverization device 10, and more preferably provided at an angle of 45 to 90 degrees. In addition, another one of the irradiating portions 30 is preferably provided at an angle of 0 to 60 degrees with respect to the vertical direction of the pulverization device 10, and more preferably provided at an angle of 0 to 45 degrees.

In addition, as shown in Fig. 1, the pulverization device 10 may include a sieve 16 for classifying the polymer block pulverized by the pulverizing portion 20. By subjecting the polymer block to the sieve 16 and extracting only the polymer block remaining on the sieve 16 (that is, excluding the polymer block which has passed through the sieve 16), it is possible to obtain a polymer block with a small variation in particle size distribution.

In addition, in the pulverization device 10, a ventilating step of ventilating the inside of the pulverizing portion 20 may be performed. Specifically, the ventilating portion 40 may ventilate the inside of the pulverizing portion 20 by sucking the air in the pulverization device 10 as shown by each arrow A in Fig. 1. By ventilating the inside of the pulverizing portion 20 by the ventilating portion 40, since the air in the vicinity of the polymer porous body during the pulverization can be replaced, the air in the vicinity of the polymer porous body during the pulverization can be efficiently ionized by the radiation radiated from the irradiating portion 30. Therefore, the electricity removing effect can be improved.

A ventilation rate of the pulverizing portion 20 by the ventilating portion 40 is preferably $1 \times 10^4$ times/h or more and $1 \times 10^7$ times/h or less. In addition, the ventilation rate of the pulverizing portion 20 by the ventilating portion 40 is more preferably $1 \times 10^5$ times/h or more and $1 \times 10^7$ times/h or less, and still more preferably $2.5 \times 10^5$ times/h or more and $1 \times 10^7$ times/h or less. The "ventilation rate" refers to a value calculated by the following expression.

$$\text{Ventilation rate (times/h)} = (\text{Air volume per hour by ventilating portion 40}$$
$$(\text{m}^3/\text{h}))/(\text{Volume of pulverizing portion 20 (m}^3))$$

The volume of the pulverizing portion 20 is preferably $1.0 \times 10^{-4}$ m$^3$ or more and $1.0 \times 10^{-2}$ m$^3$ or less, and more preferably $2.0 \times 10^{-4}$ m$^3$ or more and $1.0 \times 10^{-3}$ m$^3$ or less. The air volume by the ventilating portion 40 is preferably 25 m$^3$/h or more and 400 m$^3$/h or less, and more preferably 50 m$^3$/h or more and 200 m$^3$/h or less.

As described above, since the pulverizing portion 20 means a region where the polymer porous body can be pulverized, the "volume of the pulverizing portion 20" means "volume of the region where the polymer porous body can be pulverized". For example, in the case of the screen mill of Fig. 1, it is a volume of a region of the trapezoidal rotor inside the screen 22. In the pulverization device 10, since the electricity removing effect can be improved in a case where the above-described ventilation rate is achieved in the pulverizing portion 20 (that is, the region where the polymer porous body can be pulverized), other regions (for example, a region outside the screen 22) do not have to meet the above-described ventilation rate.

In addition, the ventilating portion 40 may suck the polymer block which has passed through the sieve 16. As the ventilating portion 40, a dust collector or the like can be appropriately used.

As described above, the pulverization device 10 includes the pulverizing portion 20 which pulverizes the polymer porous body and the irradiating portion 30 which irradiates at least a part of the pulverizing portion 20 with radiation, in which electricity is removed from the polymer porous body during the pulverization in the pulverizing portion 20 by the radiation radiated from the irradiating portion 30. Accordingly, it is possible to suppress the occurrence of burning, even in the pulverization of the polymer porous body which is easily charged and adhered due to the low density so that the burning is likely to occur.

[Polymer porous body]

Next, the polymer porous body used in the present exemplary embodiment will be described. The "polymer" is a molecule having a large molecular weight, and refers to a molecule having a structure including a large number of repetitions of a unit obtained substantially or conceptually from a molecule having a small molecular weight. Examples of the polymer include polyamines, polysaccharides, polypeptides, proteins, polyamides, polyesters, polyolefins, polyethers, and polynucleotides. The "porous body" means a solid having pores (voids) inside. The polymer porous body can be obtained, for example, by removing water from a frozen body of a polymer aqueous solution.

In addition, the polymer porous body in the present exemplary embodiment may contain a substance having biocompatibility. The "biocompatibility" means a property which does not cause a significantly harmful response such as a long-term and chronic inflammatory reaction, during contact with a living body. Examples of the substance having biocompatibility include proteins and polysaccharides. The polymer porous body in the present exemplary embodiment may contain a protein.

A size of the polymer porous body before the pulverization is preferably 0.1 mm or more and 50 mm or less. By setting the size of the polymer porous body before the pulverization within the above-described range, the time required for the pulverization can be shortened. In addition, the size of the polymer porous body before the pulverization is more preferably 0.1 mm or more and 16 mm or less. The size of the polymer porous body can be defined by a square root of a projected area of the polymer porous body, that is, a length of one side of a square having the same area as the projected are.

From the viewpoint of enhancing compatibility with the living body, a pore diameter of the polymer porous body is preferably 10 $\mu$m or more and 2500 $\mu$m or less, and more preferably 40 $\mu$m or more and 1000 $\mu$m or less. The pore diameter of the polymer porous body can be measured as a diameter of a circle (equivalent circle diameter) which has the same area as a pore portion in a case where a cut surface near the center of the polymer porous body is observed with a microscope, and can be evaluated as a median value of the equivalent circle diameters of all the pore portions within the observation area.

A density of the polymer porous body is preferably 0.01 g/cm$^3$ or more and 0.3 g/cm$^3$ or less, and more preferably 0.05 g/cm$^3$ or more and 0.1 g/cm$^3$ or less. The density of the polymer porous body can be calculated by dividing a mass of the polymer porous body by an apparent volume of the polymer porous body including internal voids.

From the viewpoint of enhancing compatibility with the living body, a void ratio of the polymer porous body is preferably 80.00% or more and 99.99% or less, and more preferably 92.01% or more and 99.99% or less. The "void ratio" refers to a value calculated by the following expression.

$$\text{Void ratio (\%)} = 100 - 100 \times \text{mass (g)} \div \text{volume (cm}^3) \div \text{true specific gravity (g/cm}^3)$$

In addition, from the viewpoint of using it as a regenerative medicine material, the polymer porous body in the present exemplary embodiment preferably contains a biodegradable polymer. Examples of the biodegradable polymer include polypeptides such as naturally derived peptides, recombinant peptides, and chemically synthesized peptides (for example, gelatin described later). In addition, examples thereof include polyglycolic acid, lactic acid/glycolic acid copolymer (PLGA), hyaluronic acid, glycosaminoglycan, proteoglycan, chondroitin, cellulose, agarose, carboxymethylcellulose, chitin, and chitosan. Among the above, it is particularly preferable that the polymer porous body in the present exemplary embodiment contains a recombinant peptide. Examples of a non-biodegradable polymer include polytetrafluoroethylene (PTFE), polyurethane, polypropylene, polyester, vinyl chloride, polycarbonate, acryl, silicone, and 2-methacryloyloxyethyl phosphoryl choline (MPC).

The type of the polypeptide such as the recombinant peptide and the chemically synthesized peptide is not particularly limited as long as it has biocompatibility and biodegradability. Examples of such a polypeptide include gelatin, collagen, elastin, fibronectin, ProNectin, laminin, tenascin, fibrin, fibroin, entactin, thrombospondin, and RetroNectin. Among these, the polymer porous body in the present exemplary embodiment preferably contains gelatin, collagen, or atelocollagen, more preferably contains natural gelatin, recombinant gelatin, or chemically synthesized gelatin, and still more preferably contains recombinant gelatin. The natural gelatin referred to herein means gelatin produced using naturally derived collagen.

Examples of the natural gelatin and the recombinant gelatin thereof include gelatins derived from an animal such as a fish and a mammal, and gelatins derived from a mammalian animal are preferable. Examples of the mammalian animal include humans, horses, pigs, mice, and rats. In a case where the polymer porous body according to the present exemplary embodiment contains the recombinant gelatin, it is more preferable that the recombinant gelatin is derived from human.

Hereinafter, an amino acid sequence constituting the polypeptide is expressed by one-letter notation (for example, "G" in a case of a glycine residue) or three-letter notation (for example, "Gly" in a case of a glycine residue) well known in the art. In addition, unless otherwise specified, "%" of the amino acid sequence of the polypeptide is based on the number of amino acid (or imino acid) residues.

(Recombinant gelatin)

Hereinafter, a recombinant gelatin with biocompatibility, biodegradability, and ability to regenerate tissue such as bone, which can be contained in the polymer porous body in the present exemplary embodiment, will be described. In the present disclosure, the recombinant gelatin means polypeptides or protein-like substances which have an amino acid sequence similar to that of gelatin produced through gene recombination technology. The following recombinant gelatin can be used as a tissue repair material which contributes to a formation of a tissue at a site by being implanted in the living body. The "tissue repair material" is not limited to a material which contributes to a formation of normal tissue normally present at the implantation site, but also includes a material which promotes a formation of abnormal tissue including scar tissue and the like. In addition, the "tissue" which can be repaired by the tissue repair material may be hard tissue such as teeth and bone, and the following recombinant gelatin is particularly suitable as a base material for bone regeneration.

The recombinant gelatin preferably has a repeat of the sequence represented by Gly-X-Y, which is characteristic of collagen. Here, a plurality of Gly-X-Y's may be the same or different from each other. In Gly-X-Y, Gly represents a glycine residue, and X and Y represent any amino acid residue other than the glycine residue. It is preferable that X and Y include a large amount of imino acid residues, that is, proline residues or oxyproline residues. A content of such imino acid residues preferably occupies 10% to 45% of the entire gelatin. A content of Gly-X-Y in the gelatin is preferably 80% or more, more preferably 95% or more, and still more preferably 99% or more with respect to the entire gelatin.

As the recombinant gelatin, for example, those described in EP1014176A2, US6992172B, WO2004/85473A, WO2008/103041A, JP2010-519293A, JP2010-519252A, JP2010-518833A, JP2010-519251A, WO2010/128672A, WO2010/147109A, and the like can be used, but the present disclosure is not limited thereto.

The recombinant gelatin preferably has a molecular weight of 2 kDa or more and 100 kDa or less, more preferably has a molecular weight of 5 kDa or more and 90 kDa or less, and still more preferably has a molecular weight of 10 kDa or more and 90 kDa or less.

In addition, from the viewpoint of biocompatibility, the recombinant gelatin preferably further contains a cell adhesion signal, and more preferably has two or more cell adhesion signals in one molecule. Examples of such a cell adhesion signal include sequences such as an RGD sequence, a YIGSR sequence, a PDSGR sequence, an LGTIPG sequence, an IKVAV sequence, and an HAV sequence. Among these, an RGD sequence is preferable, and an ERGD sequence among the RGD sequences is more preferable.

However, it is preferable that the sequence of the recombinant gelatin satisfies at least one of the following aspects (1-1) to (1-3). The recombinant gelatin may be provided alone with the following aspects (1-1) to (1-3), or may be provided with a combination of two or more aspects.

(1-1) not including a serine residue and a threonine residue
(1-2) not including a serine residue, a threonine residue, an asparagine residue, a tyrosine residue, and a cysteine residue
(1-3) not including an amino acid sequence represented by Asp-Arg-Gly-Asp

It is preferable that the recombinant gelatin has a repeating structure of A-$[(\text{Gly-X-Y})_n]_m$-B. m represents 2 to 10, preferably represents 3 to 5. A and B represent any amino acid or amino acid sequence. n represents 3 to 100, preferably represents 15 to 70 and more preferably represents 50 to 65.

It is more preferable that the recombinant gelatin is represented by Formula: Gly-Ala-Pro-$[(\text{Gly-X-Y})_{63}]_3$-Gly. In the formula, 63 pieces of X's each independently represent any amino acid residue, and 63 pieces of Y's each independently represent any amino acid residue. 3 pieces of $[(\text{Gly-X-Y})_{63}]$'s may be the same or different from each other.

It is preferable that the recombinant gelatin satisfies at least one of the following aspects (2-1) to (2-4). The recombinant gelatin may be provided alone with the following aspects (2-1) to (2-4), or may be provided with a combination of two or more aspects.

(2-1) the carbamoyl group has not been hydrolyzed.
(2-2) not having procollagen
(2-3) not having telopeptide
(2-4) a substantially pure collagen-like material prepared from a nucleic acid encoding natural collagen

From the viewpoint of high tissue repair ability, the recombinant gelatin is preferably any one of the following (A) to (C).

(A) a peptide consisting of an amino acid sequence set forth in SEQ ID NO: 1

GAP(GAPGLQGAPGLQGMPGERGAAGLPGPKGERGDAGPKGADGAPGAPG

LQGMPGERGAAGLPGPKGERGDAGPKGADGAPGKDGVRGLAGPIGPPGERGAAGLP

GPKGERGDAGPKGADGAPGKDGVRGLAGPIGPPGPAGAPGAPGLQGMPGERGAAGL

PGPKGERGDAGPKGADGAPGKDGVRGLAGPP)$_3$G (SEQ ID NO: 1)

(B) a peptide consisting of an amino acid sequence in which one or several amino acid residues in the above-described amino acid sequence set forth in SEQ ID NO: 1 are modified (for example, deleted, substituted, or added), and having biocompatibility
(C) a peptide consisting of an amino acid sequence which has a partial sequence having 80% or more sequence identity with a partial amino acid sequence consisting of 4th to 192nd amino acid residues in the above-described amino acid sequence set forth in SEQ ID NO: 1, and having biocompatibility

In the peptide defined in (B) above, the number of amino acid residues to be modified (for example, deleted, substituted, or added) varies depending on the total number of amino acid residues in the recombinant gelatin, but it is preferably 2 to 15 and more preferably 2 to 5.
In (C) above, the "sequence identity" regarding the amino acid sequences of the two kinds of peptides to be compared (that is, the peptide of (A) and the peptide of (C)) refers to a value calculated by the following expression. The comparison (alignment) of a plurality of peptides is carried out according to a conventional method so that the number of identical amino acid residues is the largest.

$$\text{Sequence identity (\%)} = [(\text{Number of identical amino acid residues})/(\text{Alignment length})] \times 100$$

In (C) above, the "partial amino acid sequence consisting of 4th to 192nd amino acid residues" corresponds to a repeating unit in the amino acid sequence set forth in SEQ ID NO: 1. The "partial sequence" corresponds to a repeating unit in the sequence (C) described above. It is sufficient that the peptide of (C) includes at least one repeating unit (partial sequence) having 80% or more sequence identity with the repeating unit in the amino acid sequence set forth in SEQ ID NO: 1, it is preferable to include two or more thereof.
In a case where the peptide of (C) includes a plurality of different repeating units, some of the plurality of repeating units may have less than 80% sequence identity with the repeating unit in the amino acid sequence set forth in SEQ ID NO: 1. However, in the peptide of (C), it is preferable that the total number of amino acid residues of the repeating unit (partial sequence) having 80% or more sequence identity with the repeating unit in the amino acid sequence set forth in SEQ ID NO: 1 is 80% or more of the total number of amino acid residues.
In addition, from the viewpoint of tissue repair ability, a sequence identity of the partial sequence in the peptide of (C) with the partial amino acid sequence consisting of 4th to 192nd amino acid residues in the amino acid sequence set forth in SEQ ID NO: 1 is preferably 90% or more, and more preferably 95% or more.
A length of the peptide defined in (C) can be 151 to 2260 amino acid residues, and from the viewpoint of decomposability after crosslinking, it is preferably 193 or more amino acid residues, and from the viewpoint of stability, it is preferably 944 or less amino acid residues. In addition, the length is more preferably 380 to 756 amino acid residues.
The above recombinant gelatin can be produced by a gene recombination technique known to those skilled in the art, and for example, it can be produced according to methods described in EP1014176A2, US6992172B, WO2004/85473A, WO2008/103041A, and the like. Specifically, a gene encoding an amino acid sequence of a predetermined recombinant gelatin is obtained, the gene is incorporated into an expression vector to prepare a recombinant expression vector, and the expression vector is introduced into a proper host to produce a transformant. The recombinant gelatin is produced by culturing the obtained transformant in an appropriate medium. Therefore, it is possible to prepare the recombinant gelatin used in the present disclosure by collecting the recombinant gelatin produced from a culture product.

By pulverizing the polymer porous body containing the recombinant gelatin, which is produced as described above, with the pulverization device 10 described in the above-described exemplary embodiment, polymer blocks having various sizes are produced, and the polymer blocks can be used as a tissue repair material, a cell scaffold, a transplant member, and the like.

The polymer porous body in the present exemplary embodiment is not limited to one containing one material such as the recombinant gelatin. For example, in addition to the recombinant gelatin, a component which promotes a reaction of the living body, such as a growth factor and a drug, and other components which can contribute to the repair or regeneration of the tissue, such as cells, may be contained. Examples of such a component include components related to bone regeneration or osteogenesis, such as a bone-inducing agent. Examples of the bone-inducing agent include bone morphogenic factor (bone morphogenic protein; BMP) and basic fibroblast growth factor (bFGF), but the bone-inducing agent is not particularly limited. In addition, the polymer porous body may be applied by mixing or compositing with an inorganic material such as hydroxyapatite.

[Polymer block]

A size of the polymer block (that is, a size of the polymer porous body after the pulverization) is preferably 0.01 mm or more and 10 mm or less. By setting the polymer block within the above-described range, a size suitable for the tissue repair material can be obtained. In addition, the size is more preferably 0.1 mm or more and 5 mm or less, and still more preferably 0.3 mm or more and 1.4 mm or less. The size of the polymer block means a size of each polymer block, and does not mean a representative value (for example, an average value, a median value, or the like) of sizes of a plurality of polymer blocks.

The size of the polymer block can be defined by the opening of the sieve in a case where the polymer block passes through the sieve. For example, in a case where the polymer block which has passed through a 1.4 mm-sieve passes through a 0.3 mm-sieve, it can be said that the polymer block remaining on the sieve is a polymer block having a size of 0.3 mm or more and 1.4 mm or less.

A shape of the polymer block is not particularly limited. For example, the shape may be particulate (granular), irregular, spherical, powdery, porous, fibrous, spindle-shaped, flat, or sheet-like. The shape is preferably particulate (granular), irregular, spherical, powdery, or porous. The "irregular" means a shape having a non-uniform surface, and examples thereof include a shape having irregularities such as rocks.

Next, the technique of the present disclosure will be described in detail with reference to Examples. The technique of the present disclosure is not limited to the following examples.

[Example 1]

(Recombinant gelatin)

In the present example, as the recombinant gelatin contained in the polymer porous body, a recombinant peptide CBE3 was used. As the CBE3, one described below was used (described in WO2008/103041A1).

Molecular weight: 51.6 kD
Structure: $GAP[(GXY)_{63}]_3G$
Number of amino acids: 571 amino acids
RGD sequence: 12 sequences
Imino acid content: 33%
Almost 100% of amino acids had a repeating structure of GXY.
The CBE3 had an ERGD sequence.

The amino acid sequence of the CBE3 did not include a serine residue, a threonine residue, an asparagine residue, a tyrosine residue, and a cysteine residue.

Isoelectric point: 9.34

Hydrophilic repeating unit ratio in polymer: 26.1%
Amino acid sequence (SEQ ID NO: 1)

GAP(GAPGLQGAPGLQGMPGERGAAGLPGPKGERGDAGPKGADGAPGAPG
LQGMPGERGAAGLPGPKGERGDAGPKGADGAPGKDGVRGLAGPIGPPGERGAAGLP
GPKGERGDAGPKGADGAPGKDGVRGLAGPIGPPGPAGAPGAPGLQGMPGERGAAGL
PGPKGERGDAGPKGADGAPGKDGVRGLAGPP)₃G

(Container used during freezing)

A cylindrical cup-shaped container made of an aluminum alloy (JIS A5056 alloy) was prepared. The container had a shape in which side surfaces and a bottom surface were closed and a top surface was open, in a case where a curved surface was regarded as the side surface. The bottom surface had a thickness of 5 mm, and an inner circumference of the bottom surface was chamfered with R2 mm. The inside of the side surface and the bottom surface was coated with a tetrafluoroethylene/hexafluoropropylene copolymer (FEP), and as a result, an inner diameter of the container was 104 mm. Hereinafter, this container will be referred to as "cylindrical container".

(Production of polymer porous body)

A solution containing the above-described recombinant gelatin was purified, and then concentrated by ultrafiltration at 30°C to 4.0% by mass. The obtained gelatin aqueous solution was freeze-dried, water for injection was added to the freeze-dried body, the temperature was raised to 37°C over 30 minutes, and the resultant was redissolved to obtain a 7.5% by mass gelatin aqueous solution again. Approximately 20 g of the gelatin aqueous solution was poured into the cylindrical container, and then 14 pieces thereof were placed on a $350 \times 634 \times 20$ mm aluminum plate precooled to approximately -35°C through a 1 mm-thick glass plate, covered with a lid, and allowed to stand for 1 hour to obtain a frozen body of the gelatin. The frozen body of the gelatin was freeze-dried using a freeze-dryer (DFR-5N-B manufactured by ULVAC, Inc.) to remove water, thereby producing a freeze-dried body. The freeze-dried body is an example of the polymer porous body according to the embodiment of the present disclosure. In addition, regarding the freeze-dried body, a void ratio was 93.9%, a pore diameter was 50 $\mu$m, and a density was 0.0748 g/cm$^3$.

The polymer porous body was manually divided into a size of 20 mm square or less, and equilibrated in an environment of 23°C and 50 %RH. The polymer porous bodies after being manually divided were arranged on a graph paper having gray ruled lines on a black background (both the black background and the gray ruled line had a lower brightness than the polymer porous body), and imaged from a direction perpendicular to the paper surface. The captured image was binarized by image processing so that a region of the polymer porous body and the rest could be separated, the region corresponding to the polymer porous body (that is, a projected area of the polymer porous body) was specified, and the square root of the projected area was calculated as the size of the polymer porous body before the pulverization. As a result, the median value was 10.6 mm, the minimum value was 6.7 mm, and the maximum value was 14.5 mm. As a software of the image processing, Imaged (manufactured by National Institutes of Health) was used.

(Pulverization of polymer porous body)

Approximately 40 g of the polymer porous body manually divided as described above was pulverized by the pulverization device 10 having the configuration shown in Fig. 1. As the pulverizing portion 20, a pulverizing portion of a screen mill (Comil U10 manufactured by Quadro Engineering) was used. As the irradiating portion 30, a soft X-ray irradiation device (L14471 manufactured by Hamamatsu Photonics K.K.) was used. As the ventilating portion 40, a dust collector (CKU-450AT-HC manufactured by CHIKO AIRTEC) was used. The pulverization was performed twice using screens 22 having different hole diameters.

In the first pulverization, a screen having a hole diameter of 0.079 inch was used as the screen 22. In this case, the pulverization was performed by operating the ventilating portion 40 such that a wind speed in the holes of the screen 22 was $8.5 \pm 0.1$ m/s, and rotating the rotary impeller 24 at a rotation speed of $1100 \pm 10$ rpm. The wind speed was measured using a digital wind speed meter (CW-60 manufactured by CUSTOM corporation). As the rotary impeller, a rake type (7A1611) was used. A suction air volume by the ventilating portion 40 was 106 m$^3$/h, and a ventilation rate was $2.6 \times 10^5$ times/h. In addition, a volume of a region of a trapezoidal rotor inside the screen 22 (that is, a region where the polymer porous body could be pulverized) was $4.1 \times 10^{-4}$ m$^3$.

In the second pulverization, a screen having a hole diameter of 0.040 inch was used as the screen 22. In this case, the pulverization was performed by operating the ventilating portion 40 such that a wind speed in the holes of the screen 22 was $10.0 \pm 0.1$ m/s, and rotating the rotary impeller 24 at a rotation speed of $2200 \pm 10$ rpm. The wind speed was measured using a digital wind speed meter (CW-60 manufactured by CUSTOM corporation). As the rotary impeller, a rake type (7A1611) was used. A suction air volume by the ventilating portion 40 was 107 m$^3$/h, and a ventilation rate was $2.6 \times 10^5$ times/h. In addition, a volume of a region of a trapezoidal rotor inside the screen 22 (that is, a region where the polymer porous body could be pulverized) was $4.1 \times 10^{-4}$ m$^3$.

After the second pulverization, polymer blocks of fractions under a sieve having an opening of 1.4 mm and on a sieve having an opening of 0.3 mm were collected, and each $89.5 \pm 3.0$ mg of the polymer blocks was packed in a 10 mL glass vial (barrel diameter: 24.3 mm).

In parallel with the above-described first and second pulverization treatments for the polymer porous body, the pulverizing portion 20 was irradiated with X-rays (soft X-rays) having a tube voltage of 15 kV from the irradiating portion 30. Specifically, as shown in Fig. 1, the pulverizing portion 20 was irradiated with the soft X-rays by a total of three irradiating portions of two irradiating portions 30A and 30B provided at an angle of 60 degrees to the left and right with respect to a vertical direction (one dot chain line) of the pulverization device 10 and one irradiating portion 30C provided on the ejecting portion 14 side.

(Evaluation of burning of polymer block)

The polymer block packed in the glass vial was observed from the bottom of the glass vial with a loupe, and the presence or absence of burning (that is, coloring) was visually inspected while shaking the glass vial. Table 1 shows the proportion of glass vials in which the burning was observed among the 30 glass vials packed with the polymer block.

(Measurement of irradiation dose)

In the configuration shown in Fig. 1, in order to evaluate an irradiation dose of the polymer porous body irradiated by the three irradiating portions 30A to 30C, the irradiation dose was measured separately from the pulverization of the polymer porous body described above. First, a $24 \times 30$ mm dental D-sensitivity instant X-ray film (DIC-100 manufactured by Hanshin Technical Laboratory, Ltd.) (hereinafter, referred to as "film") was placed at a position where the irradiation dose from the irradiating portion 30 had been known (70 $\mu$m-dose equivalent rate at 900 mm was 702 mSv/h), and the film was irradiated with the soft X-rays. The film was developed, fixed, hardened, and the like using a dedicated kit (Pusher system manufactured by Hanshin Technical Laboratory, Ltd.), and then a transmission density of the film was measured using a transmission densitometer (310 manufactured by X-Rite). Similarly, using another film, irradiation with the soft X-rays and measurement of transmission density were repeated while changing the irradiation time.

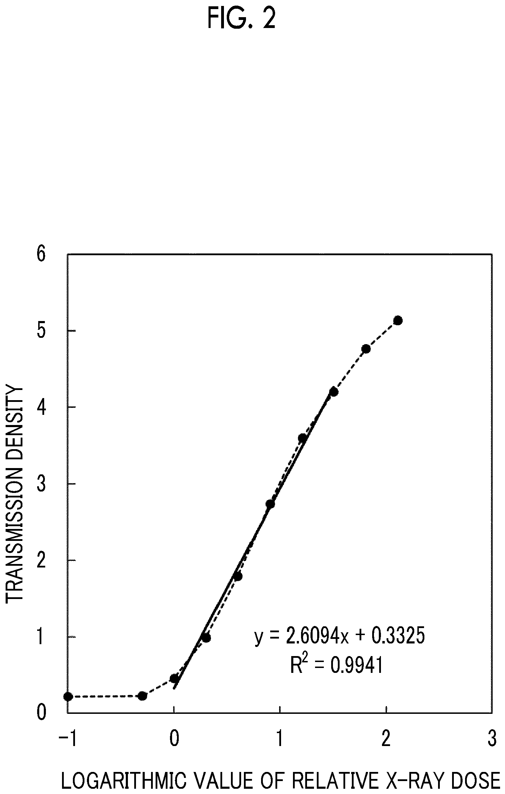

Fig. 2 shows a characteristic curve (broken line) obtained from the measurement result of the transmission density at a distance where the irradiation dose had been known. As shown in Fig. 2, the characteristic curve was represented by plotting the relative irradiation dose (the logarithmic value of the relative X-ray dose) on the horizontal axis and the transmission density on the vertical axis. By using the characteristic curve, the relative irradiation dose can be derived from the transmission density. In addition, Fig. 2 also shows an approximate straight line (solid line) which linearly approximates the linear region of the characteristic curve.

The same film as described above was disposed inside the screen 22 in the pulverizing portion 20, and irradiated with the soft X-rays for 6400 seconds by the three irradiating portions 30A to 30C. In addition, the film was developed, fixed, hardened, and the like in the same manner as described above, the transmission density of the film was measured, and then based on the approximate straight line of the characteristic curve in Fig. 2, the irradiation dose inside the screen 22 was derived from the transmission density. Table 1 shows derivation results of the irradiation dose (70 $\mu$m-dose equivalent rate) inside the screen 22. Since the irradiation dose derived above indicates the irradiation dose inside the screen 22, it can be regarded as the irradiation dose applied to the polymer porous body during the pulverization in the pulverizing portion 20.

(Measurement of electricity removing time)

In the configuration shown in Fig. 1, in order to evaluate ability of the three irradiating portions 30A to 30C to remove electricity of the pulverizing portion 20, the electricity removing time was measured separately from the pulverization of the polymer porous body described above. A 1-inch square charge plate (156P-C25X25-S3M manufactured by TREK) of a charge plate monitor (MODEL 156A manufactured by TREK) was placed inside the screen 22 in the pulverizing portion 20, and the electricity removing time until the plate voltage changed from +1000 V to +100 V was measured while being irradiated with the soft X-rays by the three irradiating portions 30A to 30C. Table 1 shows the measurement results of the electricity removing time inside the screen 22.

[Example 2]

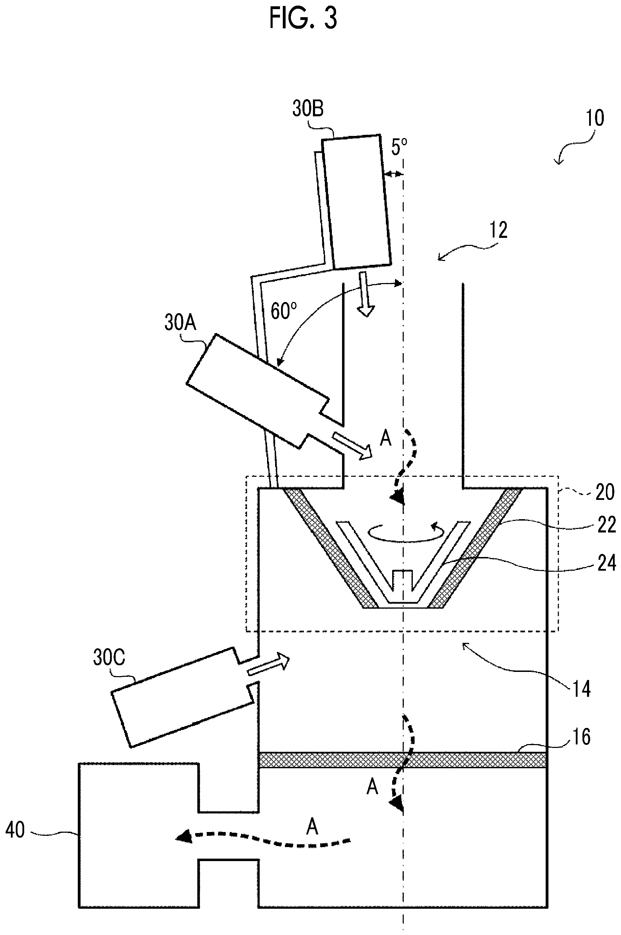

Fig. 3 shows a configuration of a pulverization device 10 according to Example 2. As shown in Fig. 3, in Example 2, the irradiation angle of the irradiating portion 30B with respect to the pulverizing portion 20 was different from that of Example 1 (see Fig. 1). Specifically, as shown in Fig. 3, the pulverizing portion 20 was irradiated with the soft X-rays by a total of three irradiating portions of two irradiating portions 30A and 30B provided at angles of 60 degrees and 5 degrees on one side with respect to the vertical direction (one dot chain line) of the pulverization device 10 and one irradiating portion 30C provided on the ejecting portion 14 side.

Other device configurations, the recombinant gelatin, the method for producing the polymer porous body, the method of pulverizing the polymer porous body, the method of evaluating the burning of the polymer block, the method of measuring the irradiation dose, and the method of measuring the electricity removing time are the same as in Example 1, so the descriptions thereof are omitted. Table 1 shows the proportion of glass vials in which the burning was observed, the result of deriving the irradiation dose inside the screen 22, and the measurement result of the electricity removing time inside the screen 22 with regard to Example 2.

[Comparative Example 1]

In order to verify the effects of the irradiating portion 30 on the electricity removal and the suppression of burning, in Example 1, the polymer porous body was pulverized without being irradiated with the soft X-rays by the irradiating portion 30. Other device configurations, the recombinant gelatin, the method for producing the polymer porous body, the method of pulverizing the polymer porous body, and the method of evaluating the burning of the polymer block are the same as in Example 1, so the descriptions thereof are omitted. Table 1 shows the proportion of glass vials in which the burning was observed with regard to Comparative Example 1.

[Table 1]

|  | Example 1 | Example 2 | Comparative Example 1 |
|---|---|---|---|
| Proportion of burning (%) | 60 | 33 | 90 |
| Irradiation dose (mSv/h) | 66 | 2 | - |
| Electricity removing time (second) | 0.6 | 1.5 | - |

As shown in Table 1, since the proportion of burning in Example 1 was 60%, the proportion of burning in Example 2 was 33%, the proportion of burning in Comparative Example 1 was 90%, it was found that the burning was suppressed in Examples 1 and 2. As a result, according to the method according to the present disclosure and the pulverization device 10, it was found that, by radiating soft X-rays, the burning which occurred in a case where the polymer porous body was pulverized could be suppressed.

In addition, in each of Examples, as the method of detecting the burning of the polymer block, an aspect of performing the visual inspection has been described, but the present disclosure is not limited thereto. The burning may be detected, for example, by detecting a colored portion of the polymer block by an image inspection. In addition, for example, the burning may be detected by detecting a change in chemical structure of the polymer block with a spectroscopic method such as infrared spectroscopy.

The disclosure of JP2021-060910 filed on March 31, 2021 is incorporated in the present specification by reference. All cited documents, patent applications, and technical standards described in the specification are incorporated by reference in the specification to the same extent as in a case where each individual cited document, patent application, or technical standard is specifically and individually indicated to be incorporated by reference. [Sequence list] International application 21F00196W1JP22013691_1.app based on International Patent Cooperation Treaty

**Claims**

1. A pulverizing method comprising:

   a pulverizing step of pulverizing a polymer porous body; and
   an electricity removing step of removing electricity from the polymer porous body during the pulverization in the pulverizing step.

2. The pulverizing method according to claim 1, wherein, in the electricity removing step, the electricity removal is performed on the polymer porous body by irradiating the polymer porous body with a radiation.

3. The pulverizing method according to claim 2, wherein the radiation is at least one of X-rays or ultraviolet rays.

4. The pulverizing method according to claim 3, wherein the radiation is X-rays having a tube voltage of a radiation source of 4 kV or more and 50 kV or less.

5. The pulverizing method according to any one of claims 2 to 4, wherein, in the electricity removing step, a 70 $\mu$m-dose equivalent rate of the radiation radiated to the polymer porous body is 1 mSv/h or more and 200 Sv/h or less.

6. The pulverizing method according to any one of claims 1 to 5, wherein, in the electricity removing step, an electricity removing time until a potential in at least a part of a pulverizing portion where the polymer porous body is pulverized changes from +1000 V to +100 V is 0.01 seconds or more and 10 seconds or less.

7. The pulverizing method according to any one of claims 1 to 6, wherein, in the pulverizing step, the polymer porous body is pulverized using a pulverizing portion which performs dry pulverization.

8. The pulverizing method according to claim 7, wherein the pulverizing portion includes a screen and a rotary impeller.

9. The pulverizing method according to claim 7 or 8, wherein the pulverizing portion is ventilated at a ventilation rate of $1 \times 10^4$ times/h or more and $1 \times 10^7$ times/h or less.

10. The pulverizing method according to claim 9, wherein, in the electricity removing step, the electricity removal is performed on the polymer porous body by irradiating at least a part of the pulverizing portion with a radiation.

11. The pulverizing method according to any one of claims 1 to 10, wherein the polymer porous body contains a protein.

12. The pulverizing method according to claim 11, wherein the polymer porous body contains the following peptide (A), (B), or (C),

    (A) a peptide consisting of an amino acid sequence set forth in SEQ ID NO: 1,
    (B) a peptide consisting of an amino acid sequence in which one or several amino acid residues in the amino acid sequence set forth in SEQ ID NO: 1 are modified, and having biocompatibility,
    (C) a peptide consisting of an amino acid sequence which has a partial sequence having 80% or more sequence identity with a partial amino acid sequence consisting of 4th to 192nd amino acid residues in the amino acid sequence set forth in SEQ ID NO: 1, and having biocompatibility.

13. The pulverizing method according to any one of claims 1 to 12, wherein a size of the polymer porous body before the pulverization is 0.1 mm or more and 50 mm or less.

14. The pulverizing method according to any one of claims 1 to 13, wherein a size of the polymer porous body after the pulverization is 0.01 mm or more and 10 mm or less.

15. A method for producing a polymer block, comprising:

    a pulverizing step of pulverizing a polymer porous body; and
    an electricity removing step of removing electricity from the polymer porous body during the pulverization in the pulverizing step.

16. A pulverization device comprising:

    a pulverizing portion which pulverizes a polymer porous body; and
    an irradiating portion which irradiates at least a part of the pulverizing portion with a radiation, wherein
    electricity is removed from the polymer porous body during the pulverization in the pulverizing portion by the radiation radiated from the irradiating portion.

17. The pulverization device according to claim 16, further comprising a ventilating portion which ventilates the pulverizing portion, wherein
a ventilation rate of the pulverizing portion by the ventilating portion is $1 \times 10^4$ times/h or more and $1 \times 10^7$ times/h or less.

# FIG. 1

# FIG. 2

# FIG. 3

## INTERNATIONAL SEARCH REPORT

| International application No. |
|---|
| **PCT/JP2022/013691** |

### A. CLASSIFICATION OF SUBJECT MATTER

*B02C 13/14*(2006.01)i; *B02C 19/18*(2006.01)i; *A61L 27/22*(2006.01)n; *A61L 27/54*(2006.01)n; *A61L 27/56*(2006.01)n; *C07K 14/00*(2006.01)i
FI: B02C19/18 B; B02C13/14 Z; B02C19/18 Z; C07K14/00; A61L27/22; A61L27/54; A61L27/56

According to International Patent Classification (IPC) or to both national classification and IPC

### B. FIELDS SEARCHED

Minimum documentation searched (classification system followed by classification symbols)

B02C1/00-B02C25/00; A61L27/22; A61L27/54; A61L27/56; B01J19/08; C07K14/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 11-320558 A (IDEMITSU PETROCHEM CO LTD) 24 November 1999 (1999-11-24) claims, paragraphs [0007]-[0073], fig. 1-6 | 1, 13-14 |
| Y | | 1, 11-15 |
| Y | WO 2017/213170 A1 (FUJIFILM CORP) 14 December 2017 (2017-12-14) claims, paragraphs [0016]-[0039], [0097]-[0100], [0134]-[0159], fig. 1-20 | 1, 11-15 |
| X | JP 48-95647 A (YOKOSAWA, Taichi) 07 December 1973 (1973-12-07) in particular, p. 1, lower left column, line 7 to p. 1, lower right column, line 8, p. 2, upper left column, lines 1-13, p. 2, upper left column, lines 19-20, drawings | 1 |
| Y | | 2-8, 13-16 |
| Y | WO 2006/068165 A1 (EISAI CO., LTD.) 29 June 2006 (2006-06-29) claims, paragraphs [0006]-[0008], [0030]-[0044], fig. 1-5 | 2-8, 13-16 |
| Y | JP 2001-257096 A (TECHNO RYOWA LTD) 21 September 2001 (2001-09-21) claims, paragraphs [0018], [0021] | 2-8, 13-16 |

✓ Further documents are listed in the continuation of Box C.    ✓ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **01 June 2022** | **14 June 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

| International application No. |
| --- |
| **PCT/JP2022/013691** |

**C.      DOCUMENTS CONSIDERED TO BE RELEVANT**

| Category* | Citation of document, with indication, where appropriate, of the relevant passages | Relevant to claim No. |
| --- | --- | --- |
| A | JP 2007-194371 A (FUJIFILM CORP) 02 August 2007 (2007-08-02) | 3-6 |
| A | JP 5-2078 A (TOSHIBA GLASS CO LTD) 08 January 1993 (1993-01-08) | 3-6 |
| A | JP 9-61400 A (MITA IND CO LTD) 07 March 1997 (1997-03-07) | 3-6 |
| A | JP 2-170827 A (AIN KK) 02 July 1990 (1990-07-02) | 1-17 |
| A | JP 57-30556 A (SHOWA TANSAN KK) 18 February 1982 (1982-02-18) | 1-17 |

Form PCT/ISA/210 (second sheet) (January 2015)

## INTERNATIONAL SEARCH REPORT

International application No.

**PCT/JP2022/013691**

**Box No. I        Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.   ☑  forming part of the international application as filed:

          ☐   in the form of an Annex C/ST.25 text file.

          ☑   on paper or in the form of an image file.

    b.   ☐  furnished together with the international application under PCT Rule 13*ter*.1(a) for the purposes of international search only in the form of an Annex C/ST.25 text file.

    c.   ☐  furnished subsequent to the international filing date for the purposes of international search only:

          ☐   in the form of an Annex C/ST.25 text file (Rule 13*ter*.1(a)).

          ☐   on paper or in the form of an image file (Rule 13*ter*.1(b) and Administrative Instructions, Section 713).

2.  ☐   In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## INTERNATIONAL SEARCH REPORT
### Information on patent family members

International application No.

**PCT/JP2022/013691**

| Patent document cited in search report | | | Publication date (day/month/year) | Patent family member(s) | | | Publication date (day/month/year) |
|---|---|---|---|---|---|---|---|
| JP | 11-320558 | A | 24 November 1999 | JP | 2007-29948 | A | |
| WO | 2017/213170 | A1 | 14 December 2017 | US | 2019/0168444 | A1 | |
| | | | | claims, paragraphs [0058]-[0105], [0097]-[0100], [0161]-[0194], [0264]-[0343], fig. 1-20 | | | |
| | | | | EP | 3470206 | A1 | |
| | | | | CN | 109311224 | A | |
| | | | | KR | 10-2019-0007446 | A | |
| JP | 48-95647 | A | 07 December 1973 | (Family: none) | | | |
| WO | 2006/068165 | A1 | 29 June 2006 | US | 2009/0123665 | A1 | |
| | | | | claims, paragraphs [0004]-[0009], [0028]-[0055], fig. 1-5 | | | |
| | | | | EP | 1839737 | A1 | |
| | | | | CN | 101080267 | A | |
| JP | 2001-257096 | A | 21 September 2001 | (Family: none) | | | |
| JP | 2007-194371 | A | 02 August 2007 | (Family: none) | | | |
| JP | 5-2078 | A | 08 January 1993 | (Family: none) | | | |
| JP | 9-61400 | A | 07 March 1997 | (Family: none) | | | |
| JP | 2-170827 | A | 02 July 1990 | US | 5080292 | A | |
| | | | | GB | 2216527 | A | |
| | | | | DE | 3906902 | A1 | |
| | | | | FR | 2628113 | A1 | |
| JP | 57-30556 | A | 18 February 1982 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014133081 A
- JP 55092667 A
- JP S55092667 A
- JP 9000176 A
- JP H9000176 A
- EP 1014176 A2
- US 6992172 B
- WO 200485473 A
- WO 2008103041 A
- JP 2010519293 A
- JP 2010519252 A
- JP 2010518833 A
- JP 2010519251 A
- WO 2010128672 A
- WO 2010147109 A
- WO 2008103041 A1
- JP 2021060910 A